# EUROPEAN PATENT SPECIFICATION

(11) **EP 1 687 037 B1**
(45) Date of publication and mention of the grant of the patent: **09.04.2008**
(21) Application number: 04812430.9
(22) Date of filing: 29.11.2004
(51) Int. Cl.: A61L 12/08, C11D 3/00

(54) **GENTLE AND ENHANCED PRESERVATIVE SYSTEMS**
SANFTE UND VERBESSERTE KONSERVIERUNGSMITTELSYSTEME
SYSTEMES DE CONSERVATION SOUPLES ET AMELIORES

(30) Priority: 01.12.2003 US 725159
(43) Date of publication of application: 09.08.2006
(73) Proprietor: BAUSCH & LOMB INCORPORATED, Rochester, New York 14604 (US)
(72) Inventor: XIA, Erning, Penfield, NY 14526 (US); HU, Zhenze, Pittsford, NY 14534 (US)
(74) Representative: Maiwald Patentanwalts GmbH
(86) International application number: PCT/US2004/039898
(87) International publication number: WO 2005/053758

(56) References cited:
- WO-A-03/053480
- US-A- 5 645 827
- US-A- 5 872 086
- US-A- 6 037 328
- US-B1- 6 495 136

## Description

### Field of the Invention:

The present invention is directed toward the use of one or more special saccharides in combination with one or more cationic polysaccharides in the manufacture of gentle and enhanced preservative systems. More particularly, the present invention is directed toward the use of compositions including one or more of said saccharides in combination with one or more cationic polysaccharides to provide gentle preservation of ophthalmic solutions and medical devices.

### Background of the Invention:

Contact lenses in wide use today fall into two general categories, hard and soft. The hard or rigid corneal type lenses are formed from materials prepared by the polymerization of acrylic esters, such as poly(methyl methacrylate) (PMMA). The gel, hydrogel or soft type lenses are made by polymerizing such monomers as 2-hydroxyethyl methacrylate (HEMA) or, in the case of extended wear lenses, by polymerizing silicon-containing monomers or macromonomers. Both the hard and soft types of contact lenses are exposed to a broad spectrum of microbes during normal wear and become soiled relatively quickly. Contact lenses whether hard or soft therefore require routine cleaning and disinfecting. Failure to routinely clean and disinfect contact lenses properly can lead to a variety of problems ranging from mere discomfort when being worn to serious ocular infections. Ocular infections caused by virulent microbes such as *Pseudomonas aeruginosa* can lead to loss of the infected eye(s) if left untreated or if allowed to reach an advanced stage before initiating treatment.

U.S. Patent Number 4,758,595 discloses a contact lens disinfectant and preservative containing a biguanide or a water-soluble salt thereof in combination with a buffer, preferably a borate buffer, e.g., boric acid, sodium borate, potassium tetraborate, potassium metaborate or mixtures of the same.

U.S. Patent Number 4,361,548 discloses a contact lens disinfectant and preservative containing dilute aqueous.solutions of a polymer; namely, dimethyldiallylammonium chloride (DMDAAC) having molecular weights ranging from about 10,000 to 1,000,000. Amounts of DMDAAC homopolymer as low as 0.00001 percent by weight may be employed when an enhancer, such as thimerosal, sorbic acid or phenylmercuric salt is used therewith. Although lens binding and concomitant eye tissue irritation with DMDAAC were reduced, it was found in some users to be above desirable clinical levels.

Despite the availability of various commercially available contact lens disinfecting systems such as heat, hydrogen peroxide, biguanides, polymeric biguanides, quaternary ammonium polyesters, amidoamines and other chemical agents, there continues to be a need for improved disinfecting and/or preserving systems. Such improved disinfecting and/or preserving systems include systems that are simple to use, are effective against a broad spectrum of microbes, are non-toxic and do not cause ocular irritation as the result of binding to the contact lens material. There is a particular need in the field of contact lens disinfection and ophthalmic composition preservation for safe and effective chemical agents with antimicrobial activity.

### Summary of the Invention:

The present invention relates to unique, gentle, self-preserving solutions such as for example but not limited to ophthalmic solutions and like solutions useful for topical application. Such self-preserving solutions may be useful for cleaning, soaking, rinsing, wetting and conditioning all types of contact lenses, , including rigid permeable contact lenses, for nasal sprays, for ear drops, for eye drops and the like. It has been found that solutions containing compositions including one or more special saccharides in combination with one or more cationic polysaccharides exhibit excellent preservative effect, while also, in the case of contact lens solution use, increasing lens wearer comfort. The saccharide/polysaccharide-containing compositions of the present invention are also useful for the preservation of ophthalmic solutions, pharmaceuticals, artificial tears, comfort drops and the like against microbial contamination.

The subject saccharide/polysaccharide-containing compositions are effective preservatives useful in the manufacture of topical solutions that are non-toxic, simple to use and do not cause ocular irritation.

Accordingly, it is an object of the present invention to provide compositions with enhanced biocidal activity useful in the manufacture of self-preserving ophthalmic systems.

Another object of the present invention is to provide a method for using compositions with enhanced biocidal activity in the preservation of medical devices.

Another object of the present invention is to provide compositions with enhanced biocidal activity useful in ophthalmic systems to preserve contact lenses.

Another object of the present invention is to provide compositions with enhanced biocidal activity useful in preserving ophthalmic systems from microbial contamination.

Another object of the present invention is to provide compositions with enhanced biocidal activity useful in ophthalmic systems for preserving contact lenses with reduced or eliminated eye irritation.

Another object of the present invention is to provide a method of making gentle compositions having biocidal activity useful in preserving ophthalmic systems.

Still another object of the present invention is to provide a method of using gentle compositions with biocidal activity as preservative agents.

These and other objectives and advantages of the present invention, some of which are specifically described and others that are not, will become apparent from the detailed description and claims that follow.

### Detailed Description of the Invention:

The compositions of the present invention can be used with all contact lenses such as conventional hard and soft lenses, as well as rigid and soft gas permeable lenses. Such suitable lenses for use with compositions of the present invention include both hydrogel and non-hydrogel lenses, as well as silicone and fluorine-containing lenses. The term "soft contact lens" as used herein generally refers to those contact lenses that readily flex under small amounts of force. Typically, soft contact lenses are formulated from polymers having a certain proportion of repeat units derived from monomers such as 2-hydroxyethyl methacrylate and/or other hydrophilic monomers, typically ,crosslinked with a crosslinking agent. However, newer soft lenses, especially for extended wear, are being made from high-Dk silicone-containing materials.

Compositions of the present invention comprise one or more special saccharides. Suitable saccharides for use in compositions of the present invention are D-glucose, methyl-α-D-gluco-pyranoside, mannitol, methyl-α-D-gluco-pyranose, turanose, galactose, trehalose and sucralose. One or more saccharides are present in the subject compositions in a total amount of from 0.001 to 10.0 percent by weight based on the total weight of the composition, but more preferably from about 0.005 to about 0.5 percent by weight.

Compositions of the present invention also comprise one or more cationic polysaccharides. The saccharide/polysaccharide-containing compositions of the present invention are useful in the production of self-preserving solutions. The self-preserving solutions are useful in preserving medical devices, pharmaceuticals, topically applied solutions and the like from microbial contamination. For example, the subject saccharide/polysaccharide-containing compositions are useful in preserving contact lens care solutions employed in cleaning, soaking, rinsing and/or wetting contact lenses. Compositions of the present invention are preferably in solution in sufficient concentration to destroy harmful microorganisms and thus preserve the solution from microbial contamination throughout the intended shelf-life of the solution.

Compositions of the present invention in solution are physiologically compatible or "ophthalmically safe" for use with contact lenses. Ophthalmically safe as used herein means that a contact lens treated with or in the subject solution is generally suitable and safe for direct placement on the eye without rinsing. The subject solutions are safe and comfortable for daily contact with the eye via a contact lens that has been wetted with the solution. An ophthalmically safe solution has a tonicity and pH that is compatible with the eye and comprises materials, and amounts thereof, that are non-cytotoxic according to ISO (International Standards Organization) standards and U.S. FDA (Food and Drug Administration) regulations. Solutions of the present invention are sterile in that the absence of microbial contaminants in the product prior to release should be statistically demonstrated to the degree necessary for such products.

As noted above, compositions of the present invention include one or more cationic polysaccharides. One or more cationic polysaccharides are present in the subject compositions in a total amount of from 0.001 to 1.0 percent by weight based on the total weight of the composition, but more preferably from about 0.005 to about 0.1 percent by weight. Suitable cationic polysaccharides for use in compositions of the present invention include for example but are not limited to variations of polyquaternium-10 such as for example Polymer JR 125^{™} (Dow Chemical Company, Wilmington, Delaware) having a 2 percent solution viscosity of 75-125 cPs and 1.5 to 2.2 percent nitrogen, Polymer JR 400^{™} (Dow Chemical Company) having a 2 percent solution viscosity of 300 to 500 cPs and 1.5 to 2.2 percent nitrogen, Polymer JR 30M^{™} (Dow Chemical Company) having a 1 percent solution viscosity of 1,000 to 2,500 cPs and 1.5 to 2.2 percent nitrogen, Polymer LR 400^{™} (Dow Chemical Company) having a 2 percent solution viscosity of 300 to 500 cPs and 0.8 to 1.1 percent nitrogen, Polymer LR 30M^{™} (Dow Chemical Company) having a 1 percent solution viscosity of 1,250 to 2,250 cPs and 0.8 to 1.1 percent nitrogen, and Polymer LK^{™} (Dow Chemical Company) having a 2 percent solution viscosity of 300 to 500 cPs and 0.8 to 1.1 percent nitrogen. The preferred cationic polysaccharide for use in the present invention is Polymer JR 125^{™} or Polymer JR 400^{™}.

The above described cationic polysaccharides comprise a cellulosic backbone derived from natural, renewable resources. Cellulose is a straight chain polymer consisting of anhydroglucose sugars linked by β-1,4-bonds. Each anhydroglucose sugar monomer has three available hydroxyl (-OH) groups. Cellulose, in its original state, has a regular, hydrogen-bonded, crystalline structure, which is not readily water soluble. The addition of the hydroxyethyl groups on the cellulose backbone alters the polymer into a water soluble, easy to use product. Quaternization of hydroxyethylcellulose results in the creation of multiple cationic sites to which the anionic surface groups of microorganisms are attracted. Due to the multiple cationic sites of the cationic polysaccharides incorporated into compositions of the present invention, antimicrobial agents commonly used in ophthalmic solutions for preservation are not necessary. Accordingly, the use of ophthalmic solutions of the present invention cause less tissue irritation, less topical toxicity, provide greater user comfort and provide a broader biocidal spectrum.

In addition to one or more cationic polysaccharides, compositions of the present invention may optionally include one or more buffers, such as aminoalcohol buffers, such as for example but not limited to ethanolamine buffers present in a total amount of from approximately 0.02 to approximately 3.0 percent by weight based on the total weight of the composition. Suitable aminoalcohol buffers include for example but are not limited to monoethanolamine (MEA), diethanolamine (DEA), triethanolamine (TEA), 2-amino-2-methyl-1,3-propanediol (AMPD), 2-dimethylamino-2-methyl-1-propanediol (DMAMP), 2-amino-2-ethylpropanol (AEP), 2-amino-1-butanol (AB) and 2-amino-2-methyl-1-propanol (AMP), but preferably MEA, DEA or TEA.

Compositions of the present invention may likewise optionally include one or more surfactants having known advantages in terms of cleaning efficacy and comfort. Surfactants may be present in the subject compositions in a total amount of from approximately 0.001 to approximately 5.0 percent by weight based on the total weight of the composition, but more preferably from about 0.1 to about 0.5 percent by weight. Suitable surfactants include for example but are not limited to polyethers based upon poly(ethylene oxide)-poly(propylene oxide)-poly(ethylene oxide), i.e., (PEO-PPO-PEO), or poly(propylene oxide)-poly(ethylene oxide)-poly(propylene oxide), i.e., (PPO-PEO-PPO),or a combination thereof. PEO-PPO-PEO and PPO-PEO-PPO are commercially available under the trade names Pluronics^{™}, R-Pluronics^{™}, Tetronics^{™} and R-Tetronics^{™} (BASF Wyandotte Corp., Wyandotte, Michigan) and are further described in U.S. Patent Number 4,820,352. Suitable surfactants for use in the present composition should be soluble in the lens care solution, not become turbid, and should be non-irritating to eye tissues.

Optionally, it may be desirable to include one or more water-soluble viscosity agents in the subject compositions. Because of the demulcent effect of viscosity agents, the same have a tendency to enhance the lens wearer's comfort by means of a film on the lens surface cushioning impact against the eye. Suitable viscosity agents include for example but are not limited to cellulose polymers like hydroxyethyl or hydroxypropyl cellulose, carboxymethyl cellulose, povidone, polyvinyl alcohol and the like. Viscosity agents may be employed in amounts ranging from about 0.01 to about 4.0 weight percent or less.

Compositions of the present invention when in solution preferably include one or more buffers, or a buffering system in addition to the aminoalcohol buffer, if any, to adjust the final pH of the solution. Suitable buffers include for example but are not limited to phosphate buffers, borate buffers, tris(hydroxymethyl)aminomethane (Tris) buffers, bis(2-hydroxyethyl)-imino-tris(hydroxymethyl)methane (bis-Tris) buffers, sodium bicarbonate, and combinations thereof. A suitable buffering system for example may include at least one phosphate buffer and at least one borate buffer, which buffering system has a buffering capacity of 0.01 to 0.5 mM, preferably 0.03 to 0.45, of 0.01 N of HCl and 0.01 to 0.3, preferably 0.025 to 0.25, of 0.01 N of NaOH to change the pH by one unit. Buffering capacity is measured by a solution of the buffers only.

The pH of lens care solutions of the present invention is preferably maintained within the range of 5.0 to 8.0, more preferably about 6.0 to 8.0, most preferably about 6.5 to 7.8.

Compositions of the present invention may likewise optionally include one or more tonicity agents to approximate the osmotic pressure of normal lachrymal fluids, which is equivalent to a 0.9 percent solution of sodium chloride or 2.5 percent glycerin solution. Examples of suitable tonicity agents include but are not limited to sodium and potassium chloride, dextrose, mannose, glycerin, calcium and magnesium chloride. These agents are typically used individually in amounts ranging from about 0.01 to 2.5 percent weight per volume and preferably, from about 0.2 to about 1.5 percent weight per volume. Preferably, the tonicity agent is employed in an amount to provide a final osmotic value of 200 to 450 mOsm/kg and more preferably between about 220 to about 350 mOsm/kg, and most preferably between about 220 to about 320 mOsm/kg.

Compositions of the present invention may also include one or more sequestering agents to bind metal ions, which in the case of ophthalmic solutions, might otherwise react with protein deposits and collect on contact lenses. Suitable sequestering agents include for example but are not limited to ethylenediaminetetraacetic acid (EDTA) and its salts. Sequestering agents are preferably used in amounts ranging from about 0.01 to about 0.2 weight percent. Surprisingly, it has been observed in formulations of the present invention that increased levels or amounts of EDTA and/or its salts in a formulation do not increase the formulation's preservative efficacy.

The compositions of the present invention are described in still greater detail in the examples that follow.

### EXAMPLE 1 - Comparison of Strength Between Saccharides (comparative example):

The preservative efficacy of various saccharides and a cationic polysaccharide were tested against one bacteria, i.e., *Staphylococcus aureus* (ATCC 6538), and against one fungus, i.e., *Aspergillus niger* (ATCC 16404). Acceptance criteria for the bacteria established that the number of viable bacteria, recovered per ml, be reduced by not less than 3.0 logs at 14 days. After the rechallenge at day 14, the concentration of bacteria shall be reduced by at least 3.0 logs at day 28. Acceptance criteria for the fungus established that the number of viable yeasts and molds, recovered per ml, remain at or below the initial concentrations within an experimental error of ± 0.5 logs within 14 days. After day 28, the concentration of mold and yeast shall remain at or below the concentrations after rechallenge within experimental error of ± 0.5 logs. Results of the saccharide/cationic polysaccharide preservative efficacy test are set forth below in Table 1.

**TABLE 1**

| **Comparison of Strength Between Saccharides** | | |
|---|---|---|
| **Saccharide** | **Log Reduction of *Staphylococcus aureus* (ATCC 6538) at 28 Days** | **Log Reduction of *Aspergillus niger* (ATCC 16404) at 28** |
| **Days** | **(Bacteria)** | **(Fungus)** |
| D-Glucose | 1.9 | 3.7 |
| Methyl-α-D-gluco-pyranoside | 1:2 | 2.9 |
| Mannitol | 2.0 | 2.4 |
| Methyl-α-D-gluco-pyranose | 1.7 | 1.6 |
| Turanose | 1.4 | 2.1 |
| Galactose | 2.2 | 1.4 |
| Trehalose | 1.3 | 2.0 |
| Polymer JR | >3.9 | 0.1 |

### EXAMPLE 2 - Preparation of Cationic Polysaccharide-Containing Test Solution (comparative example):

A cationic polysaccharide-containing sample solution for testing was prepared in accordance with the formulation set forth below in Table 2.

**TABLE 2**

| | **Test Solution** |
|---|---|
| **Ingredients W/W Percent** | **Test Solution 1** |
| Sodium Chloride | 0.135 |
| Boric Acid | 1.000 |
| EDTA | 0.050 |
| Polymer JR | 0.02 |
| pH | 7.2-7.4 |
| Osmolarity (mOsm/kg) | 180-220 |

### EXAMPLE 3 - ISO/FDA Microbial Preservative Efficacy Testing of Test Solution 1 With Five of FDA/ISO Challenge Microorganisms (comparative example):

A test solution prepared in accordance with Example 2 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of ± 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 3. As shown by the results listed in Table 3, Test Solution 1 passed the preservation efficacy test.

**TABLE 3**

| **Results of ISO/FDA Microbial Preservative Efficacy Testing Preservative Efficacy** | | |
|---|---|---|
| **ISO Agent** | **Days** | **Log Reduction of Solution 1** |
| *Staphylococcus aureus* | | |
| (ATCC 6538) | 7 | 1.9 |
| | 14 | >4.8 |
| | 21 | 1.6 |
| | 28 | >3.9 |
| *Pseudomonas aeruginosa* | | |
| (ATCC 9027) | 7 | >4.9 |
| | 14 | >4.9 |
| | 21 | >3.9 |
| | 28 | >3.9 |
| *Escherichia coli* | | |
| (ATCC 8739) | 7 | >4.9 |
| | 14 | >4.9 |
| | 21 | >3.9 |
| | 28 | >3.9 |
| *Candida albicans* | | |
| (ATCC 10231) | 7 | 2.2 |
| | 14 | 4.2 |
| | 21 | 1.3 |
| | 28 | 2.5 |
| *Aspergillus niger* | | |
| (ATCC 16404) | 7 | 1.6 |
| | 14 | 1.3 |
| | 21 | 0.6 |
| | 28 | 0.1 |

### EXAMPLE 4 - Preparation of Monosaccharide-Containing Test Solution (Comparative example):

A monosaccharide-containing sample solution for testing was prepared in accordance with the formulation set forth below in Table 4.

**TABLE 4**

| | **Test Solution** |
|---|---|
| **Ingredients W/W Percent** | **Test Solution 2** |
| Sodium Chloride | 0.135 |
| Boric Acid | 1.000 |
| EDTA | 0.050 |
| Methyl-α-D-gluco-pyranoside | 2.000 |
| pH | 7.0-7.4 |
| Osmolarity (mOsm/kg) | 260-300 |

### EXAMPLE 5 - ISO/FDA Microbial Preservative Efficacy Testing of Test Solution 2 With Five of FDA/ISO Challenge Microorganisms (comparative example):

A test solution prepared in accordance with Example 4 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of ± 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 5. As shown by the results listed in Table 5, Test Solution 2 failed the preservation efficacy test.

**TABLE 5**

| **Results of ISO/FDA Microbial Preservative Efficacy Testing Preservative Efficacy** | | |
|---|---|---|
| **ISO Agent** | **Days** | **Log Reduction of Solution 2** |
| *Staphylococcus aureus* | | |
| (ATCC 6538) | 7 | ND |
| | 14 | 1.5 |
| | 21 | 0.6 |
| | 28 | 1.2 |
| *Pseudomonas aeruginosa* | | |
| (ATCC 9027) | 7 | >4.9 |
| | 14 | >4.9 |
| | 21 | 3.5 |
| | 28 | >3.9 |
| *Escherichia coli* | | |
| (ATCC 8739) | 7 | 2.1 |
| | 14 | 3.0 |
| | 21 | 2.7 |
| | 28 | 3.3 |
| *Candida albicans* | | |
| (ATCC 10231) | 7 | 1.6 |
| | 14 | 2.6 |
| | 21 | 0.6 |
| | 28 | 0.5 |
| *Aspergillus niger* | | |
| (ATCC 16404) | 7 | 2.6 |
| | 14 | 3.4 |
| | 21 | 1.4 |
| | 28 | 2.9 |
| ND = no data | | |

### EXAMPLE 6 - Preparation of Monosaccharide and Cationic Polysaccharide-Containing Test Solution:

A monosaccharide and cationic polysaccharide-containing sample solution for testing was prepared in accordance with the formulation set forth below in Table 6.

**TABLE 6**

| | **Test Solution** |
|---|---|
| **Ingredients W/W Percent** | **Test Solution 3** |
| Sodium Chloride | 0.135 |
| Boric Acid | 1.000 |
| EDTA | 0.050 |
| Methyl-α-D-gluco-pyranoside | 2.000 |
| Polymer JR | 0.02 |
| pH | 7.2-7.6 |
| Osmolarity (mOsm/kg) | 260-300 |

### EXAMPLE 7 - ISO/FDA Microbial Preservative Efficacy Testing of Test Solution 3 With Five of FDA/ISO Challenge Microorganisms:

A test solution prepared in accordance with Example 6 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of + 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 7. As shown by the results listed in Table 7, Test Solution 3 passed the preservation efficacy test.

**TABLE 7**

| **Results of ISO/FDA Microbial Preservative Efficacy Testing Preservative Efficacy** | | |
|---|---|---|
| **ISO Agent** | **Days** | **Log Reduction of Solution 3** |
| *Staphylococcus aureus* | | |
| (ATCC 6538) | 7 | ND |
| | 14 | 3.2 |
| | 21 | 1.4 |
| | 28 | 3.2 |
| *Pseudomonas aeruginosa* | | |
| (ATCC 9027) | 7 | >4.9 |
| | 14 | >4.9 |
| | 21 | >3.8 |
| | 28 | >3.9 |
| *Escherichia coli* | | |
| (ATCC 8739) | 7 | >4.8 |
| | 14 | >4.8 |
| | 21 | >3.8 |
| | 28 | >3.8 |
| *Candida albicans* | | |
| (ATCC 10231) | 7 | 3.7 |
| | 14 | >4.9 |
| | 21 | 3.3 |
| | 28 | >3.9 |
| *Aspergillus niger* | | |
| (ATCC 16404) | 7 | 2.6 |
| | 14 | >4.7 |
| | 21 | 1.7 |
| | 28 | 2.9 |

### EXAMPLE 8 - Preparation of Monosaccharide and Cationic Polysaccharide-Containing Test Solution:

A sample solution containing a monosaccharide and a reduced amount of a cationic polysaccharide for testing was prepared in accordance with the formulation set forth below in Table 8.

**TABLE 8**

| | **Test Solution** |
|---|---|
| **Ingredients W/W Percent** | **Test Solution 4** |
| Sodium Chloride | 0.135 |
| Boric Acid | 1.000 |
| EDTA | 0.050 |
| Methyl-α-D-gluco-pyranoside | 2.000 |
| Polymer JR | 0.01 |
| pH | 7.0-7.4 |
| Osmolarity (mOsm/kg) | 260-300 |

### EXAMPLE 9 - ISO/FDA Microbial Preservative Efficacy Testing of Test Solution 4 With Five of FDA/ISO Challenge Microorganisms:

A test solution prepared in accordance with Example 8 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of ± 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 9. As shown by the results listed in Table 9, Test Solution 4 passed the preservation efficacy test showing there is no effect of the cationic polysaccharide concentration on preservation efficacy.

**TABLE 9**

| **Results of ISO/FDA Microbial Preservative Efficacy Testing Preservative Efficacy** | | |
|---|---|---|
| **ISO Agent** | **Days** | **Log Reduction of Solution 4** |
| *Staphylococcus aureus* | | |
| (ATCC 6538) | 7 | 1.3 |
| | 14 | 4.8 |
| | 21 | 1.7 |
| | 28 | >3.9 |
| *Pseudomonas aeruginosa* | | |
| (ATCC 9027) | 7 | >4.9 |
| | 14 | >4.9 |
| | 21 | 4.0 |
| | 28 | >4.0 |
| *Escherichia coli* | | |
| (ATCC 8739) | 7 | >4.8 |
| | 14 | 3.6 |
| | 21 | >3.8 |
| | 28 | >3.8 |
| *Candida albicans* | | |
| (ATCC 10231) | 7 | 1.8 |
| | 14 | 2.7 |
| | 21 | 2.4 |
| | 28 | 4.0 |
| *Aspergillus niger* | | |
| (ATCC 16404) | 7 | 2.3 |
| | 14 | 2.5 |
| | 21 | 1.9 |
| | 28 | 1.9 |

### EXAMPLE 10 - Preparation of Monosaccharide-Containing Test Solution (comparative example):

A monosaccharide-containing sample solution for testing was prepared in accordance with the formulation set forth below in Table 10.

**TABLE 10**

| | **Test Solution** |
|---|---|
| **Ingredients W/W Percent** | **Test Solution 5** |
| Sodium Chloride | 0.135 |
| Boric Acid | 1.000 |
| EDTA | 0.050 |
| D-glucose | 2.000 |
| pH | 7.0-7.4 |
| Osmolarity (mOsm/kg) | 260-300 |

### EXAMPLE 11 - ISO/FDA Microbial Preservative Efficacy Testing of Test Solution 5 With Five of FDA/ISO Challenge Microorganisms (comparative example):

A test solution prepared in accordance with Example 10 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of ± 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 11. As shown by the results listed in Table 11, Test Solution 5 failed the preservation efficacy test.

**TABLE 11**

| **Results of ISOIFDA Microbial Preservative Efficacy Testing Preservative Efficacy** | | |
|---|---|---|
| **ISO Agent** | **Days** | **Log Reduction of Solution 5** |
| *Staphylococcus aureus* | | |
| (ATCC 6538) | 7 | ND |
| | 14 | 2.0 |
| | 21 | 0.9 |
| | 28 | 1.9 |
| *Pseudomonas aeruginosa* | | |
| (ATCC 9027) | 7 | >4.9 |
| | 14 | >4.9 |
| | 21 | >3.9 |
| | 28 | >3.9 |
| *Escherichia coli* | | |
| (ATCC 8739) | 7 | ND |
| | 14 | 2.1 |
| | 21 | 1.5 |
| | 28 | 2.6 |
| *Candida albicans* | | |
| (ATCC 10231) | 7 | 0.7 |
| | 14 | 2.9 |
| | 21 | 0.8 |
| | 28 | 1.2 |
| *Aspergillus niger* | | |
| (ATCC 16404) | 7 | 2.7 |
| | 14 | 3.5 |
| | 21 | 1.6 |
| | 28 | 3.7 |

### EXAMPLE 12 - Preparation of Monosaccharide and Cationic Polysaccharide-Containing Test Solution:

A monosaccharide and cationic polysaccharide-containing sample solution for testing was prepared in accordance with the formulation set forth below in Table 12.

**TABLE 12**

| | **Test Solution** |
|---|---|
| **Ingredients W/W Percent** | **Test Solution 6** |
| Sodium Chloride | 0.135 |
| Boric Acid | 1.000 |
| EDTA | 0.050 |
| D-glucose | 2.000 |
| Polymer JR | 0.02 |
| pH | 6.0-7.0 |
| Osmolarity (mOsm/kg) | 260-300 |

### EXAMPLE 13 - ISO/FDA Microbial Preservative Efficacy Testing of Test Solution 6 With Five of FDA/ISO Challenge Microorganisms:

A test solution prepared in accordance with Example 12 above, was tested for ISO/FDA microbial preservative efficacy using five FDA/ISO challenge microorganisms, i.e., three bacteria and two fungi. Acceptance criteria established for bacteria require that the number of viable bacteria, recovered per ml, is reduced by not less than 3.0 logs at 14 days. After the rechallenge at day 14, the concentration of bacteria shall be reduced by at least 3.0 logs by day 28. Acceptance criteria established for yeasts and molds require that the number of viable yeasts and molds, recovered per ml, remain at or below initial concentrations within an experimental error of ± 0.5 logs within 14 days. After day 28, the concentration of mold and yeast shall remain at or below the concentrations after rechallenge within an experimental error of ± 0.5 logs. Results of the ISO/FDA microbial preservative efficacy testing of the subject test solution is set forth below in Table 13. As shown by the results listed in Table 13, Test Solution 6 passed the preservation efficacy test.

**TABLE 13**

| **Results of ISO/FDA Microbial Preservative Efficacy Testing Preservative Efficacy** | | |
|---|---|---|
| **ISO Agent** | **Days** | **Log Reduction of Solution 6** |
| *Staphylococcus aureus* | | |
| (ATCC 6538) | 7 | 1.2 |
| | 14 | 2.6 |
| | 21 | 1.5 |
| | 28 | 3.3 |
| *Pseudomonas aeruginosa* | | |
| (ATCC 9027) | 7 | >4.9 |
| | 14 | >4.9 |
| | 21 | >3.9 |
| | 28 | >3.9 |
| *Escherichia coli* | | |
| (ATCC 8739) | 7 | 2.2 |
| | 14 | 4.3 |
| | 21 | 1.9 |
| | 28 | 3.0 |
| *Candida albicans* | | |
| (ATCC 10231) | 7 | 2.5 |
| | 14 | 4.9 |
| | 21 | 2.0 |
| | 28 | 3.1 |
| *Aspergillus niger* | | |
| (ATCC 16404) | 7 | 2.4 |
| | 14 | 3.2 |
| | 21 | 2.9 |
| | 28 | 2.4 |

Saccharide and cationic polysaccharide containing compositions of the present invention are useful in contact lens care solutions for preserving contact lenses. A preserving amount of one or more saccharides and one or more cationic polysaccharides is an amount that will at least partially reduce the microorganism population in the formulations employed. Preferably, a preserving amount is that which will reduce the microbial burden of representative bacteria by two log orders in four hours and more preferably by one log order in one hour. Most preferably, a preserving amount is an amount that will eliminate the microbial burden on a contact lens when used according to its regimen for the recommended soaking time (FDA Chemical Disinfection Efficacy Test - July 1985 Contact Lens Solution Draft Guidelines). Unexpectedly, in the presence of one or more saccharides in combination with one or more cationic polysaccharides, a antimicrobial agent is not required to achieve effective solution preservation.

As stated above, contact lenses are preserved by contacting the lenses with a solution containing an effective amount of one or more compositions of the present composition. Although this may be accomplished by simply soaking lenses in the subject solution, greater cleaning can be achieved if a few drops of the solution are initially placed on each side of the lens, and rubbing the lens for a period of time, for example, approximately 20 seconds. The lens can then be subsequently immersed within several milliliters of the subject solution. Preferably, the lens is permitted to soak in the solution for at least four hours. The lenses are then removed from the solution, rinsed with the same or a different solution, for example a saccharide and cationic polysaccharide preserved isotonic saline solution made in accordance with the present invention, and then replaced on the eye.

Solutions containing one or more compositions of the present invention may be formulated into specific contact lens care products for use as customary in the field of ophthalmology. Such products include but are not limited to wetting solutions, soaking solutions, cleaning and conditioning solutions, as well as in-eye cleaning and conditioning solutions.

## Claims

1. A composition comprising
one or more saccharides in combination with one or more cationic polysaccharides in solution in an amount effective for solution preservation,
wherein the saccharides are present in the composition in an amount from 0.001 to 10.0 percent by weight of the composition and are selected from the group consisting of D-glucose, methyl-α-D-gluco-pyranoside, mannitol, methyl-α-D-gluco-pyranose, turanose, galactose, trehalose and sucralose, and
the cationic polysaccharides are present in the composition in an amount from 0.001 to 1.0 percent by weight of the composition.

2. The composition of claim 1 wherein said solution includes one or more buffers or buffering systems.

3. The composition of claim 1 wherein said solution includes one or more tonicity agents.

4. The composition of claim 1 wherein said solution includes one or more surfactants.

5. The composition of claim 1 wherein said solution includes one or more viscosity agents.

6. A method of preparing a composition of claim 1 comprising:
combining one or more saccharides with one or more cationic polysaccharides in an amount effective for solution preservation,
wherein the saccharides are present in the composition in an amount from 0.001 to 10.0 percent by weight of the composition and are selected from the group consisting of D-glucose, methyl-α-D-gluco-pyranoside, mannitol, methyl-α-D-gluco-pyranose, turanose, galactose, trehalose and sucralose, and
the cationic polysaccharides are present in the composition in an amount from 0.001 to 1.0 percent by weight of the composition.

7. A method of treating a contact lens comprising:
contacting a surface of a contact lens with the composition of claim 1 for a period of time suitable to eliminate a microbial burden on said contact lens.

8. A method of treating a medical device comprising:
contacting a surface of a medical device with a composition of claim 1 for a period of time suitable to eliminate a microbial burden on said medical device.

## Patentansprüche

1. Zusammensetzung umfassend ein oder mehrere Saccharide zusammen mit einem oder mehreren kationischen Polysacchariden in Lösung, in einer Menge, die wirksam ist zur Konservierung der Lösung,
wobei die Saccharide in der Zusammensetzung in einer Menge von 0,001 bis 10,0 Gew.-%, bezogen auf die Zusammensetzung, enthalten sind und ausgewählt sind aus der Gruppe bestehend aus D-Glucose, Methyl-α-D-glucopyranosid, Mannitol, Methyl-α-D-glucopyranose, Turanose, Galactose, Trehalose und Sucralose, und
die kationischen Polysacchariden in der Zusammensetzung in einer Menge von 0,001 bis 1,0 Gew.-%, bezogen auf die Zusammensetzung, enthalten sind.

2. Zusammensetzung gemäß Anspruch 1, wobei die Lösung einen oder mehrere Puffer oder Puffersysteme beinhaltet.

3. Zusammensetzung gemäß Anspruch 1, wobei die Lösung ein oder mehrere Tonizitätsagentien beinhaltet.

4. Zusammensetzung gemäß Anspruch 1, wobei die Lösung ein oder mehrere oberflächenaktive Substanzen beinhaltet.

5. Zusammensetzung gemäß Anspruch 1, wobei die Lösung ein oder mehrere Viskositätsagentien beinhaltet.

6. Verfahren zur Herstellung einer Zusammensetzung gemäß Anspruch 1, umfassend:
Vermischen von einem oder mehreren Sacchariden mit einem oder mehreren kationischen Polysacchariden in einer Menge, die wirksam ist zur Konservierung der Lösung,
wobei die Saccharide in der Zusammensetzung in einer Menge von 0,001 bis 10,0 Gew.-%, bezogen auf die Zusammensetzung, enthalten sind und ausgewählt sind aus der Gruppe bestehend aus D-Glucose, Methyl-α-D-glucopyranosid, Mannitol, Methyl-α-D-glucopyranose, Turanose, Galactose, Trehalose und Sucralose, und
die kationischen Polysaccharide in der Zusammensetzung in einer Menge von 0,001 bis 1,0 Gew.-%, bezogen auf die Zusammensetzung, enthalten sind.

7. Verfahren zur Behandlung einer Kontaktlinse, umfassend:
In Kontakt bringen einer Oberfläche einer Kontaktlinse mit der Zusammensetzung gemäß Anspruch 1 für eine Zeitspanne, die geeignet ist, um eine mikrobielle Belastung auf der Kontaktlinse zu entfernen.

8. Verfahren zur Behandlung einer medizinischen Vorrichtung, umfassend:
In Kontakt bringen einer Oberfläche einer medizinischen Vorrichtung mit der Zusammensetzung gemäß Anspruch 1 für eine Zeitspanne, die geeignet ist, um eine mikrobielle Belastung auf der medizinischen Vorrichtung zu entfernen.

## Revendications

1. Composition comprenant :
un ou plusieurs saccharides en combinaison avec un ou plusieurs polysaccharides cationiques en solution dans une quantité efficace pour la conservation de solution,
dans laquelle les saccharides sont présents dans la composition à raison de 0,001 à 10,0 pour cent en poids de la composition et sont choisis dans le groupe constitué par le D-glucose, le méthyl-α-D-gluco-pyranoside, le mannitol, le méthyl-α-D-gluco-pyranose, le turanose, le galactose, le tréhalose et le sucralose, et
les polysaccharides cationiques sont présents dans la composition à raison de 0,001 à 1,0 pour cent en poids de la composition.

2. Composition selon la revendication 1, dans laquelle ladite solution comprend un ou plusieurs tampons ou systèmes tampons.

3. Composition selon la revendication 1, dans laquelle ladite solution comprend un ou plusieurs agents de tonicité.

4. Composition selon la revendication 1, dans laquelle ladite solution comprend un ou plusieurs tensio-actifs.

5. Composition selon la revendication 1, dans laquelle ladite solution comprend un ou plusieurs agents de viscosité.

6. Procédé de préparation d'une composition selon la revendication 1, comprenant l'étape consistant à :
combiner un ou plusieurs saccharides avec un ou plusieurs polysaccharides cationiques dans une quantité efficace pour une conservation de solution,
dans laquelle les saccharides sont présents dans la composition à raison de 0,001 à 10,0 pour cent en poids de la composition et sont choisis dans le groupe constitué par le D-glucose, le méthyl-α-D-gluco-pyranoside, le mannitol, le méthyl-α-D-gluco-pyranose, le turanose, le galactose, le tréhalose et le sucralose, et
les polysaccharides cationiques sont présents dans la composition à raison de 0,001 à 1,0 pour cent en poids de la composition.

7. Procédé de traitement d'une lentille de contact comprenant l'étape consistant à :
mettre en contact une surface d'une lentille de contact avec la composition selon la revendication 1 pendant une durée appropriée pour éliminer une charge microbienne sur ladite lentille de contact.

8. Procédé de traitement d'un dispositif médical comprenant l'étape consistant à :
mettre en contact une surface d'un dispositif médical avec une composition selon la revendication 1 pendant une durée appropriée pour éliminer une charge microbienne sur ledit dispositif médical.
